# EUROPEAN PATENT APPLICATION

(11) **EP 2 237 037 A1**
(43) Date of publication of application: **06.10.2010**
(21) Application number: 10169709.2
(22) Date of filing: 12.12.2005
(51) Int. Cl.: G01N 33/543, B01L 3/00

(54) **Microfluidic device and use thereof**

(62) Divisional of application: 05816369.2
(71) Applicant: GYROS PATENT AB, 751 83 Uppsala (SE)
(72) Inventor: Holmquist, Mats, 192 73, Sollentuna (SE); Jesson, Gerald, 741 42, Knivsta (SE)
(74) Representative: Widén, Björn

(57) **Abstract**

A microfluidic device comprises one or a plurality of microchannel structures each of which comprises in the downstream direction a reaction microcavity RM (105) and a capture microcavity CM (106) in which there is a solid phase exposing an affinity binder (B). There are also a) two or more inlet units (108,109,110) that are in downstream communication with RM (105) and CM (106), and b) one or more inlet units (107,111) that are in downstream fluid communication with CM (106) but not with RM (105). The microfluidic device may further comprise a mixing function between said two or more inlet units (108,109,110) and said RM (105) which mixing function may at least partly coincide with RM (105).

## Description

### TECHNICAL FIELD

The present invention relates to a microfluidic method for determining the amount of an analyte in a liquid sample by the use of a reactant (Re) that is capable of binding to the analyte (An) by affinity, and to a microfluidic device in which the method can be carried out. The method is either a competitive receptor-ligand assay, e.g. a competitive immunoassay, or a catalytic assay, e.g. an enzymatic assay.

### DRAWINGS

- **Figures 1**: gives a generalized flow scheme of a microchannel structure that can be used in the present invention.
- **Figures 2a-c**: illustrate a preferred microchannel structure. Figure 2c is the same as figure 2b except that the numbers given are dimensions in mm.
- **Figure 3**: illustrates a set of microchannel structures that has been used in the competitive immunoassays described in the experimental part.
- **Figure 4**: gives the result of experiment 1 (quantitative immunoassay of substance P).
- **Figure 5**: gives the result of experiment 2 (quantitative immunoassay of substance NPY).
- **Figure 6**: illustrates schematically the methodology used in experiment 2

Reference numbers in the drawings are given with three digits. The first digit refers to the number of the drawing and the two next digits to particular items.

### BACK-GROUND TECHNOLOGY

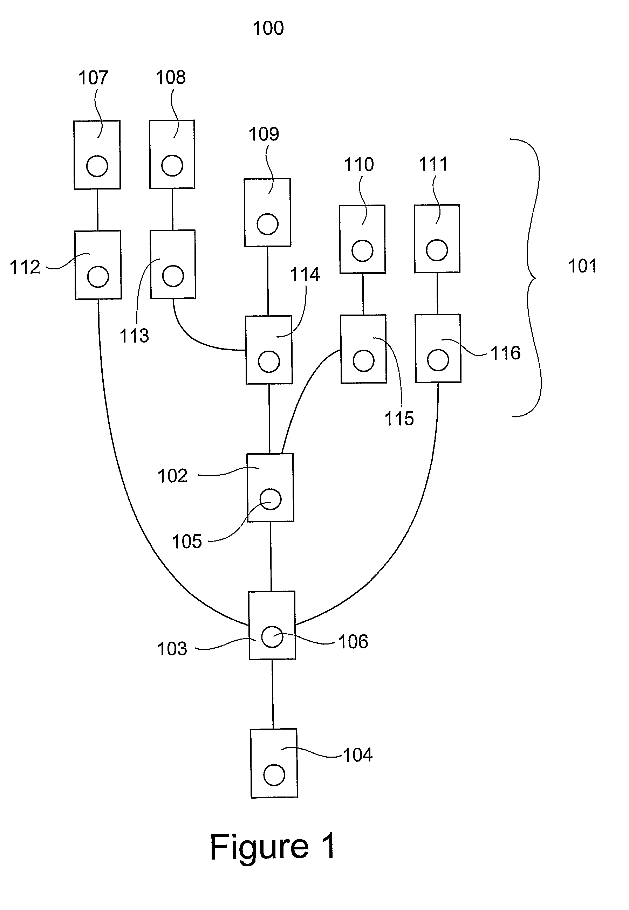

Microfluidic devices are well known in the field. Asingle device typically comprises a plurality of microchannel structures. The flow scheme of a typical microchannel structure **(100)** is illustrated in figure 1 and comprises in the downstream direction:
(A) an inlet and sample preparation arrangement (ISA) **(101),**
(B) an optional reaction zone (RZ) **(102),**
(C) a measuring zone (MZ) **(103),** and
(D) an outlet and waste arrangement (OWA) **(104).**

One or more distinct microcavities **(105** and **106),** possibly containing a solid phase, are typically present in each ofRZ **(102)** and MZ **(103).** ISA **(101)** typically contains one, two or more inlet units (IU) **(107-111)** and may optionally also contain one or more reactant or sample transformation units (RTU) **(112-116).** The individual IUs and RTUs may be associated with the same part or with separate parts of a microchannel structure. Further details of microfluidic devices/microchannel structures are discussed under the heading "Micofluidic devices".

The inventive method is based on two earlier known basic assay protocols each of which utilizes an affinity reactant Re for the formation of a product P:
a) Competitive/inhibition affinity assays (= ligand-receptor assays). The reactant (Re) is an affinity counterpart (anti-An) to both the analyte (An) and to an analogue to the analyte (An-analogue). The product P comprises the affinity complex Re---An-analogue in which Re and An-analogue bind directly to each other.
b) Catalyst based assays, i.e. assays that utilize a catalytic system that converts a substrate S to the product P via a transient affinity complex that comprises substrate S (= Re) and one or more other components of the catalytic system. One of these other components is the analyte. In the transient complex the analyte and substrate S (Re) bind directly (Re---analyte) or indirectly (Re---B---analyte) to each other. B is then an affinity counterpart to both the analyte and Re (anti-An,Re) and may contain/consist of one or more affinity reactants that also are components of the catalytic system.

The protocols (a) and (b) when applied to microfluidic devices comprises the steps of:
(i) providing a product P that has been obtained according to (a) or (b) above in immobilized form within the measuring zone MZ **(103)** of the microchannel structure **(100)** of a microfluidic device,
(ii) measuring the amount of product P in the measuring zone MZ **(103).**
The conditions for obtaining the product P have been selected such that the amount of product P correlates with the amount of analyte in the sample. The correlation means that the amount of analyte in the sample can be calculated (step (iii)) from the measured value for product P obtained in step (ii). Calculation can be made by comparing a measured value with the corresponding value(s) for known amounts (standards, standard curves etc), for instance. Conditions include proper selection of reagents including their relative amounts, pH, ion strength etc.

Further details are given in WO 9853311 (Gamera Biosciences), WO0079285 (Gamera Biosciences), WO 02075312 (Gyros AB), WO 04083109 (Gyros AB), WO 04083108 (Gyros AB), WO 03093802 (Gyros AB), WO 05072872 (Gyros AB), WO 0410926 (Gyros AB) etc.

Immobilizable/insolubilizable affinity reactants have previously been suggested in macroscale competitive and non-competitive assays. See for instance US 4,469,796 (Axén et al), US 4,298,685 (Burroughs & Wellcome), US 3,839,153 (Schuurs et al), EP 0048357 (Engvall et al) etc. Corresponding use of this type of reactants in microfluidic devices has been mentioned in WO 02075312 (Gyros AB) and WO 04083109 (Gyros AB).

An important subaspect of the invention relates to kinase assay protocols, i.e. a particular kind of protocol (b) above.

Kinases are enzymes that catalyse the transfer of a phosphate group from ATP to a substrate. Their assays are based on the quantification of phosphorylated product or the depletion of ATP. The phosphorylation of substrate occurs at serine, threonine or tyrosine in a specific manner depending on the kinase. Most current kinase assay methods require antibodies, radioactive labeling or indirect measurement of secondary reactions to measure transfer of the phosphate group. For an overview of marketed kinase assays see "How to choose an in vitro kinase assay" (Drug Discovery and development, March 2004, 59-64).

Protein kinases have key roles in a large number of immune-related diseases, such as cancer, immune diseases, diabetes etc. This has led to extensive efforts to develop kinase inhibitors that are potent as drugs in the treatment of these diseases. Accordingly, kinase assays have played a key role in screening for suitable drug candidates that are kinase inhibitors.

Accordingly the most potent aspects of kinase assays according to the invention relate to the determination/detection of kinase activity, possibly in order to screen for kinase inhibitor activity of a compound.

All patents and patent applications cited above and elsewhere in this specification are incorporated in their entirety by reference.

### DEFINITIONS

The term "heterogeneous" in the context of the above-mentioned assay protocols means that
a) product P during the assay is partitioned to a solid phase in an amount that is correlated with the amount of analyte in an original sample, and
b) the solid phase containing the immobilized product P, and the liquid phase are separated from each other, i.e. either the liquid phase or the solid phase is removed from the reaction mixture in which immobilization of product P is taking place.

An analogue of an affinity reactant is capable of competing with and/or inhibiting affinity binding between the affinity reactant concerned and an affinity counterpart to this reactant, e.g. an An-analogue competes with and/or inhibits affinity binding of the analyte to the reactant Re. This typically means that the analogue contains the identical or similar (= "same") binding site as the reactant of which it is an analogue.

The terms "dissolved", soluble or "solubilized" are used interchangeable and means that the reactants concerned and product P are true solutes or are in suspended form, for instance firmly attached to suspended particles. The liquids discussed herein are typically aqueous, preferably with water as one of the main components of a liquid used (e.g. ≥ 30 % w/w).

The term "fluid communication" between two parts of a microchannel structure means that liquid is intended to be transported between the parts.

### OBJECTS OF THE INVENTION

The inventors and their colleagues have during some years been searching for good microfluidic assays according to protocol (a) and (b). During this process it has been found that the protocols so far suggested often have been inefficient in performance in one or more respects, e.g. specificity, accuracy, reproducibility, time per run, handling, robustness etc. This inefficiency has been particularly pronounced when going down in volume into nl-volumes. Inefficiencies have occurred for certain analytes, certain kinds of samples, certain kind of reagents etc and often varied between analytes, kinds of samples, kinds of reagents etc. New generic assay protocols and new designs of generic microchannel structures would be welcomed and beneficial for a successful commercial introduction of microfluidics in clinical diagnostics.

It would thus be advantageous to have a generic protocol and/or microchannel structure that permit: 1) a high degree of freedom in the selection of incubation times for the steps leading to product P, 2) a generic capturing function on a solid phase, 3) a capturing function that does not rely upon the affinity of the reactants used to form product P, 4) simple use of low affinity reactants, such as antibodies, in the formation of the product P, 5) multiplex analysis of several analytes in the same reaction mixture; and/or 6) a minimum of steps for incubation and/or washing and/or conditioning.

It would further be advantageous with a generic protocol and/or a generic microchannel structure that easily could be adapted to both competitive affinity assays of protocol (a) and catalytic assays of protocol (b), such as kinase assays.

The "ideal" kinase assay should meet the following criteria:
a) non-radioactive, b) compatible with both peptide and protein substrates, c) possibility to handle substances that give background fluorescence, d) no negative impact from the reagents used for substrate conversion on the measurement (e.g. luciferase used for measurement may be inhibited by reactants that are present during phosphate transfer), e) possibility to work at high ATP concentrations, f) non-antibody based. There is thus a general desire in the field to set up protein kinase assays in which two, three, four, or five of these desires can be met.

Inefficiency of an assay protocol often depends on poor and/or undesired interactions between dissolved reactants and immobilized affinity reactants, such as immobilized analyte analogues and immobilized components of catalytic systems, or simply between reactants and inner walls of a microchannel structure. The latter kind of undesired interactions typically becomes particularly significant and more prominent in nl-volume based microfluidic assays (≤ 10x10³ nl, such as ≤ 5x10³ nl or ≤ 1x10³ nl or ≤ 0.5 x10³ nl) than in systems utilizing larger volumes (≤ 1 µl, such as ≤ 10 µl or ≥ 20 µl). nl-volumes primarily contemplate volumes that contain the immobilized reactant or a soluble reactant such as the analyte or an analytically detectable reactant.

The primary object of the invention is to present solutions that give one or more of the above-mentioned advantages and/or to fully or partly overcome the problems discussed above.

### THE INVENTION

The present inventors have realized that in order to comply with the primary object of the invention it is appropriate to physically separate the location where the complex (product P) to be measured is formed from the location where the immobilization of product P takes place. By doing so it has been possible to achieve further improvements by selecting immobilization reactions that are fast and/or have equilibrium that suggest non-reversibility under the conditions used.

The first aspect of the invention is a method for determining the amount of an analyte in a sample by utilizing a microfluidic device of the type generally outlined in the introductory part with the proviso that the formation (step (i)) and measurement (step ii) of product P are physically separated from each other, for instance in RZ **(102)** and MZ **(103),** respectively. This includes that step (i) in some variants means that product P in dissolved form is obtained outside the device.

The inventive method typically also contains a calculating step (step (iii)) after the measuring step.

The main characteristic features of the invention comprise:
(A) using for the formation of product P a reactant combination that:
   I) for competitive receptor-ligand assay protocols comprises that:
      a) An-analogue exhibits an immobilizing tag, and anti-An (= Re) exhibits an analytically detectable group, or
      b) An-analogue is a covalent conjugate between an analyte moiety, and an analytically detectable group in the form of a label with the label and the analyte moiety being linked together covalently via a bridge that preferably is hydrophilic, and anti-An (= Re) exhibits an immobilizing tag, and
   II) for catalytic assays comprises that substrate S (= Re): a) comprises an immobilizing tag, or b) is devoid of an immobilizing tag but contains a group that is transformable to such a tag during the course of the protocol, and
(B) the product P is obtained in dissolved form and exhibits the immobilizing tag and an analytically detectable group,
(C) the measuring zone MZ **(103)** contains a predisposed solid phase in a capture microcavity (CM) **(106,),** and
(D) step (ii) comprises the substeps of:
   a) immobilizing product P via the immobilizing tag to the solid phase, and
   b) measuring the amount of product P immobilized to the solid phase.

In certain variants of competitive receptor-ligand assays neither An-analogue nor Re comprises an immobilizing tag. Instead they have a taggable group that either during or after the formation of the complex (= An-analogue---Re) can be transformed to a group that exposes the immobilizing tag, for instance by reaction with an affinity reactant that comprises both the immobilizing tag and a moiety that is the affinity counterpart to the taggable group. In these latter variants the product P formation step includes also introduction of the immobilizing tag on the taggable group. Similarly applies also to catalytic variants (II above) in which substrate S comprises the transformable group.

### STEP (i): PROVIDING PRODUCT P IN THE MEASURING ZONE (MZ)

### Competitive affinity assays (ligand-receptor assays)

The analyte and its affinity counterpart Re (anti-An) are reactants that typically are members of an affinity pair, such as antigen/hapten and antibodies, complementary nucleic acids, hormone and hormone receptor, lectin and carbohydrate, Ig-constant region binding proteins/polypeptide and Ig-constant region etc. A member of an affinity pair that is used as a reactant in a competitive affinity assay is typically unchanged during the affinity reactions utilized in a protocol (except for conformational changes and the fact that the reactant becomes part of an affinity complex). This kind of reactants includes also derivatives, fragments and synthetic mimetics etc that exhibit cross-reactive affinity with a member of an affinity pair. The An-analogue comprises a) a first moiety that is related to the analyte and b) a second moiety that is an immobilizing tag or an analytically detectable group, such as a label. Reactant Re accordingly contains an analytically detectable group if the An-analogue contains an immobilizing tag, and an immobilizing tag if the An-analogue contains an analytically detectable group. The analytically detectable group may in both variants be a label. The analyte is typically a low molecular weight compound, for instance with a molecular weight ≤ 25,000 dalton such as ≤ 15,000 dalton or ≤ 10,000 dalton or ≤ 1,000 dalton. A lower limit is typically 100 dalton.

The affinity complex Re---An-analogue which is part of the product P can be obtained in a number of ways, for instance:
a) In a first variant a limited amount of Re (= anti-An) is saturated with An-analogue to give the complex Re---An-analogue, whereafter An-analogue in a second step is displaced from this complex by the analyte. The remaining amount of Re---An-analogue complex will correlate with the amount of analyte used for displacement and also with the unknown amount of analyte in the original sample. The second step will thus be part of the product P formation step and take place in RZ **(102),** or outside the device. In a typical variant two defined liquid aliquots containing known amounts of Re (= anti-An) and An-analogue, respectively, are mixed and incubated with each other in ISA **(101)** or outside the device (1^{st} step). A defined volume of this mixture is after incubation introduced into RZ **(102)** where it is mixed and incubated with a defined liquid aliquot containing the analyte (2^{nd} step).
b) In a second variant analyte and An-analogue are allowed to compete with each other for binding to a limiting amount of Re (= anti-An) in one single step. The amount of complex Re---An-analogue formed will correlate with the amount of analyte added and also with the unknown amount of analyte in the original sample. This single step will thus be part of the product P formation step and take place in RZ **(102),** or outside the device. The reaction is started by mixing liquid aliquots containing analyte, An-analogue and Re (= anti-An), for instance by mixing three aliquots, each of which contains one of the reactants, in a mixing function associated with the reaction microcavity (RM) **(105),** or outside the device. In a typical variant, liquid aliquots containing analyte and An-analogue, respectively, are mixed in a mixing unit within ISA **(101)** or outside the device. The mixture is subsequently introduced into RZ **(102)** where a defined volume of the mixture is mixed with a liquid aliquot containing Re and incubated in the reaction microcavity (RM) **(105).**
c) In a third variant analyte is allowed to bind to a non-limiting known amount of Re (= anti-An) in a first step whereafter in a subsequent second step remaining binding sites on Re (anti-An) are saturated with a non-limiting amount of An-analogue, for instance a slight excess that is sufficient to saturate the remaining sites. The amount of complex Re---An-analogue is in the second step formed in an amount that will correlate with the amount of added analyte and with the unknown amount of analyte in the original sample. Thus the second step will be part of the product P formation step and take place in RZ **(102),** or outside the device. In a typical variant two defined liquid aliquots containing of Re (anti-An) and the analyte, respectively, are mixed and incubated with each other in ISA **(101)** or outside the device (1^{st} step). A defined volume of this mixture is after incubation introduced into RZ **(102)** where it is mixed and incubated with a defined liquid aliquot containing the non-limiting amount of An-analogue (2^{nd} step).
d) In a fourth variant the analyte and An-analogue is allowed to compete with each other for binding to a non-limiting of Re (anti-An) in one single step. It can be arranged so that in the initial phase of the reaction the amount of the complex Re---An-analogue reflects the amount of added analyte in the starting mixture. This single step can be initiated by mixing in the same manners as suggested for alternative b) above.

If the product P formation step takes place within the device then the preceding step(s) may take place either in ISA **(101)** of the device or outside device.

If An-analogue or Re comprises a taggable group instead of the immobilizing tag as discussed above, transformation of the taggable group to an immobilizing group is part of the product formation step and takes place either outside the device or within RZ **(102),** for instance simultaneously with or subsequently to the formation of An-analogue---Re in RM (**105**) or in a separate reaction microcavity (not shown) that is downstream of RM **(105).**

### Catalytic assays

Catalytic systems primarily contemplate biocatalytic systems, for instance enzymatic systems that are based on enzymatically active proteins or synthetic variants thereof. Components of a catalytic system can be illustrated with catalysts, substrates, cosubstrates, cofactors, cocatalysts, inhibitors, promoters, activators etc including also other effector molecules that are capable of affecting substrate conversion. For enzymatic systems this corresponds to enzymes, substrate, cosubstrates, coenzymes, cofactors, inhibitors, promotors etc. The term catalytic system also contemplates coupled systems comprising a catalytic substrate conversion, i.e. systems linked together such that the product of one system is a component/reactant of another system, e.g. the product or the substrate of an initial catalytic substrate conversion may be a component/affinity reactant of a ligand-receptor affinity reaction system or a reactant in a pure organic/inorganic reaction system.

The analyte is in this variant of the invention one of the components of the catalytic system at issue (except for not being the product formed by the system). The analyte is different from the substrate (substrate S = Re) that is used for the introduction of the immobilizing tag on product P.

The analyte can in principle be any of the components of the catalytic system used. As already discussed the analyte may bind directly or indirectly by affinity to substrate S (= Re). The binding sites for a substrate and the binding site for an effector molecule may be physically separated on the same molecule, for instance on an enzyme.

The catalytic system should be capable of transforming substrate S to a product P that comprises both the immobilizing tag and an analytically detectable group. The detectable group is selected such that it makes it possible to discriminate product P from other entities having tags with the same immobilizing characteristics as product P. Thus the catalytic system should be able to produce product P from a substrate S that
a) contains the analytically detectable group but not the immobilizing tag by introducing the immobilizing tag, or
b) contains the immobilizing tag but not the analytically detectable group by introducing the analytically detectable group, or
c) neither contains the analytically detectable group nor the immobilizing tag by introducing both the tag and the group.
Alternatives a) and b) typically require single catalytic system while alternative c) typically requires coupled systems (at least one system for the label and at least one for the tag).

Catalytic systems in the form of enzymatic systems may be selected amongst: 1) Oxidoreductases (dehydrogenases, oxidases etc), 2) Transferases, 3) Hydrolases (esterases, carbohydrases, proteases etc), 4) Lyases, 5) Isomerases, and 6) Ligases.

Appropriate catalytic systems to which the invention may be applied are hydrolases for which a number of substrates are known that enzymatically can be transformed to products that have fluorescence or luminescence properties that the corresponding substrates do not have. It would be relatively simple to design this kind of substrates with an immobilizing tag that is retained in the product, for instance by biotinylation or haptenylation. In an analogous manner, a protein kinase is capable of introducing phospho groups on serine and/or threonine and/or tyrosine in protein and polypeptide substrates containing anyone of these amino acid residues. In the case this phospho group is present on a product comprising an immobilizing tag, such as biotin, the phospho group can be used as a detectable group and measured in step (ii.b) by the use of the appropriate anti-phospho antibody after immobilization via the immobilizing tag in step (ii.a). Alternatively this kind of phospho group may be used as an immobilizing tag that permit immobilization in step (ii.a) to a solid phase exposing an appropriate antibody specific for a phosporylated amino acid residue or an IMAC group (= metal chelate group), and using the "immobilizing tag" (biotin) as an analytically detectable affinity group that is measured in step (ii.b) by the use of for instance labeled anti-biotin antibody or some other biotin-binding substance, such as streptavidin or neutravidin.. Other transferases are likely to be useful in the analogous manner by the use of the specific groups introduced. Ligases may be used in combination with two different substrate molecules that are capable of being ligated by a ligase provided that one of the substrate molecules contains a detectable group while the other one contains an immobilizing tag.

The formation of product P in the catalytic variant of the invention comprises, for instance, simultaneous mixing of all of the reactants (= catalytic components) necessary for starting the catalytic reaction within a mixing unit of RZ **(102)** and incubating in a reaction microcavity RM **(105)** of the same RZ **(102).** This mixing typically means that two or more liquid aliquots, each of which contains a single reactant or a combination of reactants, are mixed in the mixing unit. One of the aliquots typically comprises at least the analyte. Alternatively, components of the catalytic system used are premixed and possibly allowed to bind to each other in mixing units and reaction microcavities upstream RZ **(102),** i.e. in an RTU **(114)** of ISA **(101),** with the proviso that mixtures containing combinations that by themselves leads to formation of product P are only prepared in RZ **(102).** Premixing and/or preincubating in this context contemplate that one or more liquid aliquots containing the remaining components for an active substrate conversion is mixed with the premixture in RZ **(102).** Suitable remaining components are for instance substrate S, one or more imperative effectors, the catalyst as such etc.

Premixing and/or preincubation steps, possibly in combination with the actual product P formation step may take place outside the microfluidic device.

In the case the catalytic system is a coupled system, for instance as described above, the complete system, if possible, can be applied in the reaction microcavity RM **(105)** of RZ **(102)** as illustrated in the outline of kinase assays in experiment 3. Alternatively, individual parts of coupled catalytic system may be applied consecutively with the first catalytic substrate conversion step and subsequent parts of the product P formation step taking place within RZ **(102),** typically with said substrate conversion taking place within RM **(105)** and subsequent part steps in one or more reaction microcavities (not shown) that are downstream of RM **(105)** but within RZ **(102).** Applied to the kinase assay of experiment 3 this means that the enzymatic part and the immunological part of the coupled system is carried out in separate reaction microcavities with the catalytic part in RM **(105)** and the immunological part in a reaction microcavity downstream RM **(105)** but still within RZ **(102).**

Product P is after its formation transported into in the measuring zone (MZ) **(103)** irrespective of being formed within or outside the device. This applies to both competitive and catalytic assay protocols

### STEP (ii) MEASURING PRODUCT P IN THE MEASURING ZONE (MZ)

This step comprises two substeps: a) immobilization of the product in the capture microcavity (CM) **(106),** and b) measurement of the amount of product P that becomes immobilized in CM **(106).**

### Step (ii.a) and immobilizing tag and a reactive counterpart (anti-tag group))

Immobilization may take place under static conditions or more preferably under flow conditions. The term "static conditions" in this context contemplates that the flow through CM **(106)** is halted during immobilization, typically during more than 90 % of the period of time used for contact between product P and the solid phase in CM **(106).** The term "flow conditions" contemplates that the reaction mixture containing product P is flowing continuously through CM **(106)** during immobilization, typically for more than 90 % of the period of time used for contact between product P and the solid phase in CM **(106).** Flow conditions typically leads to a better concentrating of the immobilized product to the inlet part of the solid phase. Typically the flow rate used should give a residence time for the reaction mixture of ≥ 0.010 seconds such as ≥ 0.050 sec or ≥ 0.1 sec with an upper limit that typically is < 2 hours such as < 1 hour. Illustrative flow rates are within 0.001-10,000 nl/sec, such as 0.01-1000 n1/sec or 0.01-100 nl/sec or 0.1 - 10 nl/sec. These intervals may primarily be useful for solid phase volumes in the range of 1-1,000 nl, such as 1-200 nl or 1-50 nl or 1-25 nl. Residence time is the time it takes for a liquid aliquot to pass the solid phase. Optimization typically will require experimental testing.

The liquid flow through the solid phase can be driven by in principle any kind of forces, for instance electrokinetically or non-electrokinetically forces as described elsewhere in this specification. Centrifugal force created by spinning the microfluidic device, possibly combined with capillary force are preferred.

Immobilization of product P takes place via the immobilizing tag that typically should give selectivity in the immobilization. Constituents (in the liquid in which product P has been formed), which would disturb in subsequent steps, for instance by containing a group that is detectable in the same manner as the analytically detectable group in product P (see below), shouls thus become immobilized to a much lesser degree than product P.

In order to accomplish the desired selectivity in step (ii.a), the solid phase typically contains a firmly attached reactive group that is counterpart (anti-tag) to the immobilizing tag on product P. During step (ii.a) product P, will thus become firmly attached to the solid phase via bonds created between the immobilizing tag and the anti-tag group. A possible excess of a reactant, which contains the immobilizing tag, will also become immobilized (e.g. An-analogue or its affinity counterpart (= anti-An = Re) or substrate S (=Re)).

The anti-tag group are in many variants in molar excess in CM **(106)** compared to the immobilizing tag that is present in the reaction mixture containing product P (e.g. coming from RM **(105)).** The excess may be > 2-fold, such as > 5-fold or > 25-fold or > 50 fold or even more, such as 500-fold or 5000-fold. This does not exclude that the anti-tag group on the solid phase may be in a deficient amount compared to the immobilizing tag, for instance in the case one would like to reduce the signal from the analytically detectable group, such as the label. Such deficient amount may be < 0.5-fold, such as < 0.2-fold or < 0.04-fold.

An immobilizing tag and its reactive counterpart (anti-tag) are called an immobilizing binding pair. There are two main kinds of such pairs: a) covalently immobilizing pairs, and b) affinity immobilizing pairs.

The use of a covalently immobilizing pair typically means that the anti-tag group is a chemically reactive group that is capable of forming a covalent bond with the immobilizing tag. Typical pairs includes among others so called soft electrophilic groups versus the corresponding soft nucleophilic groups. Soft electrophilic groups are α-halo carbonyl (in particular α-iodo carbonyl), α,β-alkene carbonyls (such as in N-substituted maleimide structures), disulfides (-S-S-) (in particular so called reactive disulfides), and asymmetrically oxidized disulfides (such as -S-SOₙ- (where n is 1 or 2)), etc. The corresponding soft nucleophilic group is primarily the thiol group (-SH). So called hard nucleophilic groups and the corresponding hard electrophilic groups may also be used. Hard electrophilic groups are imido carbonate, oxirane, carbonate etc. Hard nucleophilic groups are hydroxy, amino etc. The electrophilic group is attached to the solid phase in the most typical immobilizing pairs of this kind.

Selective covalent immobilization of affinity complexes in competitive affinity assays is described in US 4,469,796 (Axén et al). Immobilization by the use of oxidized disulfides is described in US 5,807,997 (Batista)

The use of an affinity immobilizing pair means that the anti-tag group is an affinity counterpart to the tag. The immobilizing tag is typically called affinity binder or simply binder (B) and its counterpart on the solid phase is called-ligand (L). This kind of pair should be selected such that, except for the desired affinity binding, the members of the pair should be essentially devoid of other binding abilities during the conditions used.

Preferred affinity immobilizing pairs (L and B) typically have equilibrium constants (K_{L--B} = [L][B]/[L--B]) that are ≤ 10 times or ≤ 10² times or ≤ 10³ times larger than the corresponding constant for streptavidin and biotin. This typically will mean constants that roughly are ≤ 10⁻¹³ mole/l, ≤ 10⁻¹² mole/l, ≤ 10⁻¹¹ mole/l and ≤ 10⁻¹⁰ mole/l, respectively. This does not exclude that also immobilizing binding pairs for which the corresponding constants are > 10⁻¹¹ mole/l can be used, for instance between 10⁻⁶ and 10⁻¹¹ mole/l, such as within the interval 10⁻⁷ to 10⁻¹¹ mole/l or 10⁻⁸ to 10⁻¹⁰ mole/l. These ranges refer to values obtained by a biosensor (surface plasmon resonance) from Biacore (Uppsala, Sweden), i.e. with either B or L immobilized to a dextran-coated gold surface while the other is in dissolved form.

It is believed that it is advantageous that the ligand L has two or more binding sites for the binder B, and/or binder B has one, two or more binding sites for the ligand L (or vice versa).

Particular examples of affinity immobilizing pairs are a) streptavidin/avidin/ neutravidin and a biotinylated reactant (or vice versa), b) antibody and a haptenylated reactant (or vice versa), c) an IMAC group and an IMAC-binding motif (i.e. an oligopeptide containing single or a sequence of histidyl, cysteinyl, phosphorylated aminoacyl etc residues), anti-species specific or anti-class specific antibodies and Ig species specific and Ig class specific determinants etc. Sequence in this context comprises two, three, four, five, six or more residues. Typical aminoacyls that may be phosphorylated are threonine, tyrosine and serine.

The preference is to select L and B amongst biotin-binding compounds and streptavidin-binding compounds, respectively, or vice versa. Streptavidin and neutravidin are believed to work best as affinity ligand L.

The affinity ligand L should be attached more firmly to the solid phase than the binder B is affinity bound to the affinity ligand L in step (ii.a). This typically means covalent attachment to the solid phase of the affinity ligand L although also adsorptive bonds involving electrostatic attraction, van-der Waals bonding etc may be used.

Step (ii.a) typically also comprises a separating step in which the solid phase with its immobilized product P is physically separated from excess reactants. Separation is typically accomplished by transporting the liquid phase with dissolved components through the solid phase while immobilizing product P. Components that comprises the tag will be retained by the solid phase while other dissolved components will pass through. This separation step may comprise one or more washing steps in which one or more aliquots of washing liquid are passed through the solid phase to make the removal/separation more efficient.

### Step (ii.b). Measuring the amount of product P in the measuring zone MZ - detectable groups.

This substep means that the amount of the analytically detectable group that is present in the immobilized product P is measured. The reactants and the immobilizing tag have been selected such that the solid phase will contain negligible amounts, if any, of the detectable group that has been immobilized via other routes than via product P.

The detectable group is typically an affinity group, a signal-generating group or the like. The group may be synthetically introduced on a reactant and is then called a label and the labeled reactant is a labeled conjugate. Alternatively the detectable group is natively present in the reactant. The analytically detectable group may also be created during the formation of product P.

Examples of detectable affinity groups are biotin, haptens, class-, subclass- or species-specific determinants on antibodies etc. Biotin and hapten are examples of compounds that typical are used as labels in the invention, i.e. in conjugates. Detectable affinity groups that are present in a product P require as a rule a secondary detectable reactant that has affinity for the detectable group on product P. This secondary reactant comprises a detectable group that should be distinguishable from the detectable group in product P but otherwise is selected amongst the same candidates as the detectable group in product P. The secondary reactant preferably is a conjugate and comprises a label that is catalytic, such as a component of a catalytic system, or otherwise is capable of generating a measurable signal (chromophor, fluorophor, luminophor, radioactive etc). The detectable group in the secondary reactant may also be a detectable affinity group in which case there is required also a tertiary reactant comprising a detectable group and a moiety that is affinity counterpart to the detectable group of the secondary reactant. Secondary, tertiary etc reactants of this kind are preferably introduced via one or more inlet units **(107,111;210)** that are in downstream fluid communication with CM **(106,206)** without passing RM **(105,205).** In less preferred variants the introduction may be via other inlet units, e.g. the introduced liquid passing through RM **(105,205)** in a similar manner as for inlet units **(108-110;207-209).**

The most important signal-generating analytically detectable groups are synthetically introduced into a reactant. The generated signal is typically some kind of radiation, such as visible light at a certain wavelength, fluorescence, luminescence, radioactivity etc.

A signal-generating detectable group may be catalytically active and the detectable group, then is a component of catalytic systems such as an enzymatic system. The label is typically a catalyst, a co-catalyst, substrate, inhibitor, activator or the like which for enzymatic system will be enzyme, co-enzyme, substrate, co-substrate, cofactor, inhibitor, activator or the like as described for catalytic assays above. Catalytically active label typically convert a substrate to a product that may be in dissolved or insoluble form. The substrate differs from the product with respect to one or more detectable properties that are used when measuring the amount of product P.

A signal-generating group may alternatively be non-catalytic and then typically is a group that is capable of emitting radiation and/or interacting with incoming radiation. These labels are typically selected amongst chromophors, fluorophors, luminophors, radioactive groups etc. In this context luminophors includes chemiluminophors, bioluminophors and the like.

The analytically detectable group maybe releasable from the immobilized product P. A released label that is soluble (in dissolved form) may be transported downstream and measured in a separate detection microcavity that is part of the measuring zone MZ **(103).** See for instance WO 02075312 (Gyros AB) for microfluidic devices and US 4,231,999 (Carlsson et al) for larger static systems. Similarly also applies for catalytically active labels that result in soluble products. In the case of insoluble products that are analytically detectable, measurement many times can take place directly on the solid phase in CM **(106)**

### Step (iii). Calculation step.

This step is performed according to established principles as outlined elsewhere in this specification. The main objective is to receive a measure that enables comparison of analyte occurrence and activity between different samples or with standards. Amounts in this context contemplate concentrations, such as in absolute and/or relative figures (for instance relative to a standard or to a non-analyte component(s) of the sample etc), and includes binding activity, enzymatic activity etc.

### Conjugates

The term "conjugate" primarily refers to man-made/man-designed covalent conjugates between two different affinity binders or between one affinity binder and a label. This kind of conjugates may be obtained by chemically or recombinantly linking the moieties of the conjugates together.

In preferred variants of the invention the An-analogue and/or Re (anti-An or substrate S) are in the form of conjugates. These kinds of conjugates comprise
A) a first moiety that relative to the analyte in the competitive receptor ligand assays can act as a) an An-analogue, or b) an affinity counterpart (anti-An) to the analyte, or in the catalytic assays can act as substrate S, and
B) a second moiety that can act as a) an immobilizing tag, or b) a label.
As suggested elsewhere in this specification conjugates may also be used in other process steps of the invention.

The two moieties of the conjugates are covalently held together by a bridge that typically is hydrophilic or amphiphilic. Typical bridges provide a distance of at least 5 atoms between the moieties. That the bridge is hydrophilic or amphiphilic contemplates that the ratio between the number of heteroatoms (nitrogen and oxygen) and the number of carbon atoms in the bridge is ≥ 0.1, such as ≥ 0.2, and typically is ≤ 1. Instable structures, such as peroxy groups and groups containing a carbon directly binding a hydroxyl or an amino group and an additional oxygen and nitrogen, etc are in most cases not present. In the preferred conjugates the second moiety or the bridge is preferably a polymeric carrier for the first moiety or for both in the case the carrier is a bridge. Suitable carriers have polymeric structure and exhibit for instance a plurality of structures selected amongst peptide structures, carbohydrate structures, nucleic acid structures etc, for instance, or is a synthetic polymer. It follows that suitable polymers should comprise a plurality of heteroatom-containing hydrophilic groups, such as hydroxy and/or amido and/or ether. Preliminary experiments have shown that the bridges and polymeric carriers described in this paragraph, in particular polymeric carriers exhibiting polypeptide or polysaccharide structure, might be very useful in conjugates that are used in competitive receptor-ligand assays. Enzymes are typical polymeric.carriers that can be used both as a carrier and the second moiety (= label). Serum albumin and other water-soluble/hydrophilic polymers that do not participate in intended reactions of an assay, for instance, are a typical polymeric carriers that can be used as a bridging molecule between the first and second moieties. It seems beneficial for An-analogue conjugates to contain a carrier structure as discussed herein for carrying a plurality of An-moiteies, in particular if the other moiety is a label. This kind of conjugates is preferably combined with low-molecular analytes as discussed elsewhere in this specification.

### MICROFLUIDIC DEVICES

A microfluidic device is defined as a device in which one or more liquid aliquots that contain reactants and have volumes in the µl-range are transported and processed in microchannel structures that have a depth and/or width that are/is in the µm-range. The µl-range is ≤ 1000 µl, such as ≤ 25 µl, and includes the nl-range that in turn includes the pl-range. The nl-range is ≤ 5000 nl, such as ≤ 1000 nl. The µl-range is ≤ 5000 µl, such as ≤ 1000 pl. The µm-range is ≤ 1000 µm, such as ≤ 500 µm.

A microfludic device typically contains a plurality of the microchannel structures described above, i.e. has two or more microchannel structures, such as ≥ 10, e.g. ≥ 25 or ≥ 90. The upper limit is typically ≤ 2000 structures.

Different principles may be utilized for transporting the liquid within a microchannel structur. Inertia force may be used, for instance by spinning the disc as discussed in the subsequent paragraph. Other useful forces are electrokinetic forces and non-electrokinetic forces other than centrifugal force, such as capillary forces, hydrostatic pressure, pressure created by one or more pumps etc.

The microfluidic device typically is in the form of a disc. The preferred formats have an axis of symmetry (Cₙ) that is perpendicular to or coincides with the disc plane, where n is an integer ≥ 2, 3, 4 or 5, preferably ∞ (C_{∞}). The disc thus may have various polygonal forms such as rectangular. The preferred sizes and/or forms are similar to the conventional CD-format, e.g. sizes in the interval from 10% up to 300 % of a circular disc with the conventional CD-radii (12 cm). If the microchannel structures are properly designed and oriented, spinning of the device about a spin axis that typically is perpendicular or parallel to the disc plane may create the necessary centrifugal force for causing parallel liquid transport within the structures. In the most obvious variants at the priority date, the spin axis coincides with the above-mentioned axis of symmetry.

For preferred centrifugal-based variants, each microchannel structure comprises one upstream section that is at a shorter radial distance than a downstream section (from the spin axis). The capture microcavity (CM) **(106,206),** for instance, is then typically at a radial position intermediary to two such sections.

In preferred microchannel structures, capillary force is used for introducing liquid through an inlet port up to a first capillary valve whereafter centrifugal force or some other non-passive driving means is applied for overcoming the resistance for liquid flow at the valve position. The same kind of forces/driving means is also used for overcoming capillary valves at other positions.

In order to facilitate efficient transport of liquid between different functional parts, inner surfaces of the parts should be wettable (hydrophilic), i.e. have a water contact angle ≤ 90°, preferably ≤ 60° such as ≤ 50° or ≤ 40° or ≤ 30° or ≤ 20°. These wettability values apply for at least one, two, three or four of the inner walls of a microconduit. The wettability or hydrophilicity, in particular in inlet arrangements, should be adapted such that an aqueous liquid will be able to fill up an intended microcavity/microconduit by capillarity (self suction) once the liquid has started to enter the cavity/microconduit. A hydrophilic inner surface in a microchannel structure may comprise one or more local hydrophobic surface breaks (water contact angle ≥ 90°). Such a break may wholly or partly define a passive/capillary valve, an anti-wicking means, a vent to ambient atmosphere etc. Contact angles refer to values at the temperature of use, typically +25°C, and are static. See WO 00056808, WO 01047637 and WO 02074438 (all Gyros AB).

### MICROCHANNEL STRUCTURES

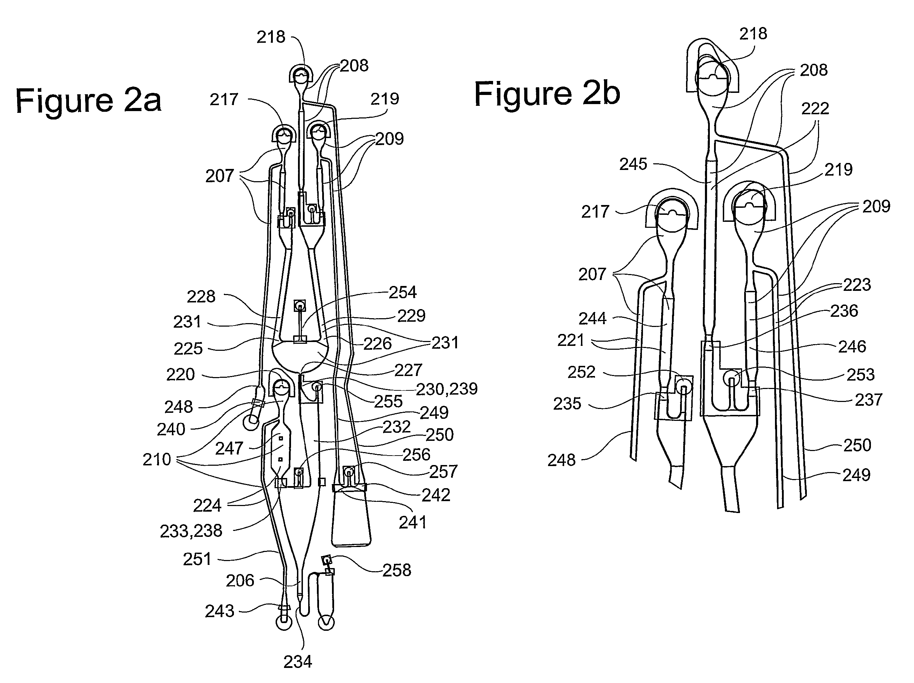

Suitable microchan nel structures will be described based on the preferred structure illustrated in figures 2a and b and follow the general outline given in the introductory part.

### Inlet and sample preparation arrangement (ISA)

The inlet arrangement ISA **(101)** has one, two or more inlet units (IU) (**107-111,207-210)**, and possible also a reactant or sample transformation unit (RTU) **(112-116).** Two or more of the inlet units **(107-111,207-210)** may be in downstream communication with the same part or with different parts of a microchannel structure, for instance with the same microcavity or with different microcavities. Compare IU **(210)** with IUs **(207-209).**

An inlet unit **(107-111,207-210)** is in the upstream direction typically in fluid communication with ambient atmosphere and therefore comprises an inlet port/opening **(217-220)** for liquid. The inlet unit may also have a volume-defining unit **(221-224)** in which a liquid aliquot to be transported downstream is metered. In the downstream direction an inlet unit ICT **(107-111,207-210)** is in fluid communication with the reaction microcavity RM **(105,205),** the capturing microcavity CM **(106,206)** and/or some other microcavity in MZ **(103),** and/or a microcavity in an RTU **(112-116).**

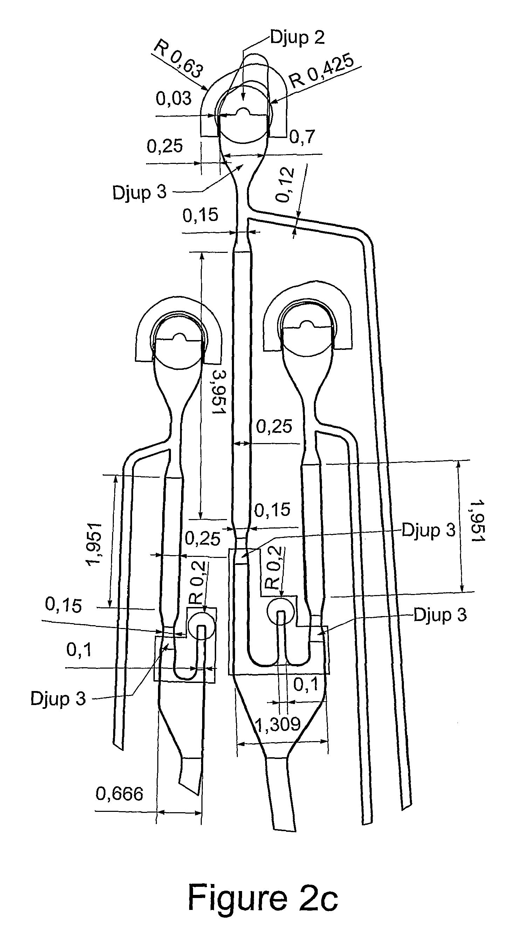

Metering of liquid volumes in volume-defining units **(221-224)** is preferably based on the over-flow principle. A detailed description of the preferred volume-defining unit given in figure 2 is given in WO 02074438 (Gyros AB).

The corresponding inlet unit in several microchannel structures (subgroup) of a device may be linked together in a liquid distribution manifold that is common for the microchannel structures. See WO 02074438 (Gyros AB).

A reactant and sample transformation unit (RTU) **(112-116)** comprises functional units that are necessary for transforming samples and reactants that are introduced into the structure to forms that are required by the product P formation step in RM **(105,205)** or other process step as described in this specification. Typical such functional units are separation units for removing undesired particles, mixing units, volume-defining units, reaction microcavities etc. Different RTUs are connected to the same or to different parts of a microchannel structure, for instance one RTU may be connected to RM **(105,205)** and another one to CM **(106,206),** respectively. An RTU **(112-116)** is typically connected to ambient atmosphere via an inlet unit **(107-111)** for liquid.

Suitable inlet units including liquid distribution manifolds are described in WO 04058406 (Tecan), WO 9853311 (Camera Biosciences), WO0187486 (Tecan Trading), WO 02074438 (Gyros AB), WO 03018198 (Gyros AB), WO 9958245 (Amersham Pharmacia Biotech). Separation units are described in WO 02074438 (Gyros AB) and WO 03018198 (Gyros AB). Reaction microcavities may be of the same type as in RZ **(102)** (see below).

### Reaction zone RZ

A reaction zone RZ **(102)** comprises one or more reaction microcavities used for carrying out the product P formation step. The most upstream of them RM **(105,205)** is shown in figure 2 and is used for the formation of the complex An-analogue---Re in an amount that is related to the analyte (protocol (a)) or for the substrate S conversion step (protocol (b)). Other reaction microcavities in RZ **(102),** if present, are located downstream of RM **(105,205)** and are typically used for further processing the resulting reaction mixture obtained in RM **(105,205),** for instance removal of entities that otherwise may have a negative impact on the measurement or immobilization in MZ **(103)** and derivatization of the product formed in RM **(105,205).** Derivatization in this context may comprise that a product comprising an analytically detectable affinity group is reacted with a labeled conjugate that comprises an affinity counterpart to this particular detectable group, or that a reactant comprising an immobilizing tag is reacted with the product obtained in RM to introduce the tag on the product.

A reaction microcavity RM **(105,205)** in RZ **(102)** comprises one, two or more inlet openings **(225-226)** and at least one outlet opening **(227).** Each inlet opening **(225-226)** is connected to an inlet microconduit **(228-229)** and the outlet opening **(227)** to an outlet micro conduit **(230).** The inlet microconduit **(228-229)** is in the upstream direction in fluid communication with a vent function and/or one or more inlet units **(108-110,207-209)** for liquid as discussed above possibly via various parts of ISA **(101)** or via a mixing function **(231)** used for mixing the liquid aliquots used in the product P formation step in RM **(205).** The outlet microconduit **(230)** is in the downstream direction in communication with CM **(206)** of MZ **(103).**

RM **(205)** is typically associated with a mixing unit (231), which for instance comprises a mechanical mixer or is based on mixing during liquid transport in a mixing microconduit that ends in a collection microcavity (that typically also is used as a reaction microcavity) as recently suggested for centrifugal based microfluidic devices) (US 6,582,663, WO 00079285, US 6,527,432, US 20020097632; US 20030152491, WO 01087487 (all Tecan Trading); WO 02074438, WO 03024598, WO 05094976 (all Gyros AB)) and for non-centrifugal based microfluidic devices (US 6,379,929 (Univ. Mich.)). This mixing unit may fully or partly coincide with RM **(205)** and/or with one or more of the inlet microconduits **(228-229)** of RM **(205).** See figure 2. Compare also the mixing function in WO 02074438 (unit 2) (Gyros AB) and WO 03018198 (units A and B (Gyros AB). The variant illustrated in figure 2 utilizes back-and-forth transport in the mixing microconduit of the aliquots to be mixed. See WO 05094976 (Gyros AB).

One or **more** of the additional reaction microcavities in RZ may or may not be associated with a mixing function as discussed for the mixing function **(231)** associated with RM **(205).**

### The measuring zone MZ

MZ (103) comprises at least a capture microcavity CM **(106,206)** that contains a solid phase to which product P is to be immobilized. The measuring zone MZ **(103)** also comprises a detection microcavity DM that may coincide with CM **(206)** (as in figure 2) or is placed downstream CM (not shown). A detection microcavity DM that coincides with CM **(206)** is used when the analytically detectable group that generates the signal to be measured is retained on the solid phase. A detection microcavity DM that is separate from CM **(106,206)** is primarily used when detecting/measuring the immobilized product P utilizes a soluble signal generating substance that is formed in, released from, or passed through and partly consumed in CM **(206).** This kind of detection microcavity DM may comprise a solid phase for capture of the signal generating substance and detection/measurement on the solid phase, or is devoid of a solid phase meaning that detecting/measuring of the signal-generating substance is taking place in solution. See for instance WO 0275312 (Gyros AB). Between CM and DM there is preferably a liquid split (router, branching) permitting waste liquids to pass to a waste function without passing through DM. See for instance WO 0274438 (Gyros AB), WO 05032999 (Gyros AB), US 2005032999 (Gyros AB) and publications cited in these applications.

As discussed above the solid phase in the CM **(106,206)** typically comprises an anti-tag group that is used for the immobilization of product P.

CM **(106,206)** is typically a straight microconduit that may or may not be widening and/or narrowing in the flow direction. The capture microcavity CM **(106,206)** typically has at least two upwardly directed microconduits **(232-233)** and one or more microconduits **(234)** that at least initially are directed downwards. One of the upwardly directed microconduits is a liquid inlet microcoduit **(232)** through which product P is introduced into CM **(106,206)** and thus is in upstream fluid communication with the reaction microcavity RM/reaction zone RZ **(105,205/102),** if present. The remaining upwardly directed microconduits **(233)** are typically used for venting purposes and/or for separate introduction of liquid aliquots that are not needed in the product P formation process. If an upwardly directed microconduit of the latter kind is used for inlet of liquid into CM **(106,206)** it is typically in upstream fluid communication with an inlet unit IU and/or reactant and sample transformation unit RTU, such as inlet ports and volume-defining units, respectively, that is separate from the corresponding units for liquid aliquots containing reactants used in the formation of product P. In preferred variants CM is part of an Y-shaped structure as illustrated in figure 2 with two upwardly directed liquid inlet microconduits **(232-233)** and one downwardly directed outlet microconduit **(234)** and with CM **(106,206)** located downstream of the merging of the two inlet microconduits.

The CM **(106,206)** typically has at least one cross-sectional dimension in the µm-range. The volume is typically in the nl-range. These ranges are defined elsewhere in this text. Since the solid phase and the capture microcavity CM are mutually coinciding the same ranges also apply to the solid phase.

The outlet microconduit **(234)** from CM **(206)** is typically designed as a restriction microconduit that is capable of creating a pressure drop that is larger than the total interchannel variation in flow resistance emanating from positions upstream and downstream the microconduit. See further WO 03024598 (Gyros AB).

The solid phase may be a porous bed, i.e. a porous monolithic bed or a bed of packed particles that may be porous or non-porous. Alternatively, the solid phase may be an inner wall of CM **(206).** The term "porous particles" is given in WO 02075312 (Gyros AB).

Suitable particles to be used in a porous bed are spherical or spheroidal (beaded), or non-spherical. Appropriate mean diameters for particles are typically found in the interval of 1-100 µm with preference for mean diameters that are ≤ 5 µm, such as ≥ 10 µm or ≥ 15 µm and/or ≤ 50 µm. Also smaller particles can be used, for instance with mean diameters down to 0.1 µm. Diameters refer to the "hydrodynamic" diameters. Particles may be monodisperse (monosized) or polydisperse (polysized) in the same meaning as in WO 02075312 (Gyros AB).

The base material of a solid phase may be made of inorganic and/or organic material. Typical inorganic materials comprise glass and typical organic materials comprise organic polymers. Polymeric materials comprise inorganic polymers, such as glass and silicone rubber, and organic polymers that may be of synthetic or biological origin (biopolymers).

The term "hydrophilic" in the context of a porous bed contemplates a sufficient wettability of the surfaces of the pores for water to be spread by capillarity all throughout the bed when in contact with excess water (absorption). The expression also means that the inner surfaces of the bed that is in contact with an aqueous liquid medium during the immobilization shall expose a plurality of polar functional groups which each has a heteroatom selected amongst oxygen and nitrogen, for instance. Appropriate functional groups can be selected amongst hydroxy groups, ethylene oxide groups, amino groups, amide groups, ester groups, carboxy groups, sulphone groups etc, with preference for those groups that are essentially uncharged independent of pH, for instance within pH 2-12.

If the base material of a solid phase material is hydrophobic or not sufficiently hydrophilic, e.g. is based on a styrene (co)polymer, the surfaces that are to be in contact with an aqueous liquid may be hydrophilized to contain polar functional groups of the same type as discussed above.

The solid phase is typically predisposed to CM **(106,206)** by which is meant that the solid phase is introduced into the microchannel structure before the actual assay protocol is performed. Predisposing may thus take place before the device is offered for sale, for instance during the manufacture of the device. See for instance WO 04083108 (Gyros AB) that among others describe predisposed solid phases exposing a firmly attached affinity counterpart to an immobilizing tag.

CM **(106,206)** may be connected to one or more separate or joined inlet units (207,**210** and **208** + **209,** respectively) for liquid. These inlet units are in the downstream direction uniquely associated with CM **(106,206)** or with positions in the measuring zone that are upstream of CM. Their connections to the measuring zone **(103)** thus do not pass through RM (105) or RZ (102). Between this kind of inlet units and the measuring zone there may be a RTU (not shown). This RTU may contain one or more units selected from separation units, volume-defining units, reaction microcavities, mixing units etc as generally discussed above for ISA **(101)** and RTU **(116).** This kind of inlet units is preferably used for the introduction of reagents and washing liquids used in the measuring step. See above.

The microchannel structure typically contains a number of valve functions and other functions controlling the liquid transport. Typically there is a valve **(235-238,239,240-243)** at the outlet opening of each volume-metering microcavity **(244-247),** at the outlet opening **(227)** of each reaction microcavity **(205)** and in the downstream part of each overflow microconduit **(248-251).** These valves may be mechanical or non-mechanical including non-closing valves such as passive valves or capillary valves. Preferred capillary valves are based on abrupt changes in at least one cross-sectional dimension and/or an abrupt non-wettable break (hydrophobic break) in the wettability of an otherwise wettable micro conduit. Different kinds of valves that can be used in the invention have been discussed in WO 9721090 (Gamera Bibsciences), WO 9807189 (Gamera Biosciences), WO 9853311 (Gamera Biosciences), WO 02074438 (Gyros AB), WO 03018198 (Gyros AB), WO 04103890 (Gyros AB); WO 04103891 and US SN 10/849,321 (Gyros AB) etc.

The microchannel structure typically also contains a number of vents **(252-258)** for inlet and/or outlet of ambient atmosphere in order to promote smooth liquid transport without creation of local overpressures and gas bubbles.

At the appropriate positions within a microchannel structure there may also be so called anti-wicking functions to prevent undesired transport in the edges by wicking, for instance between different functionalities and/or in inlet units to render losses by evaporation difficult. Capillary valves **(235-238,239,240-243)** based on local non-wettable breaks in wettable surfaces may often work as anti-wicking functions

Details of vents, inlet ports, anti-wicking functions etc are discussed in WO 9721090 (Gamera Biosciences), WO 9807189 (Gamera Biosciences), WO 98533111 (Camera Biosciences), WO 02074438 (Gyros AB), WO 03018198 (Gyros AB), WO 04103890 (Gyros AB); WO 04103891 and 20050042770 (Gyros AB) etc.

### EXPERIMENTAL PART

### MICROFLUIDIC DEVICE

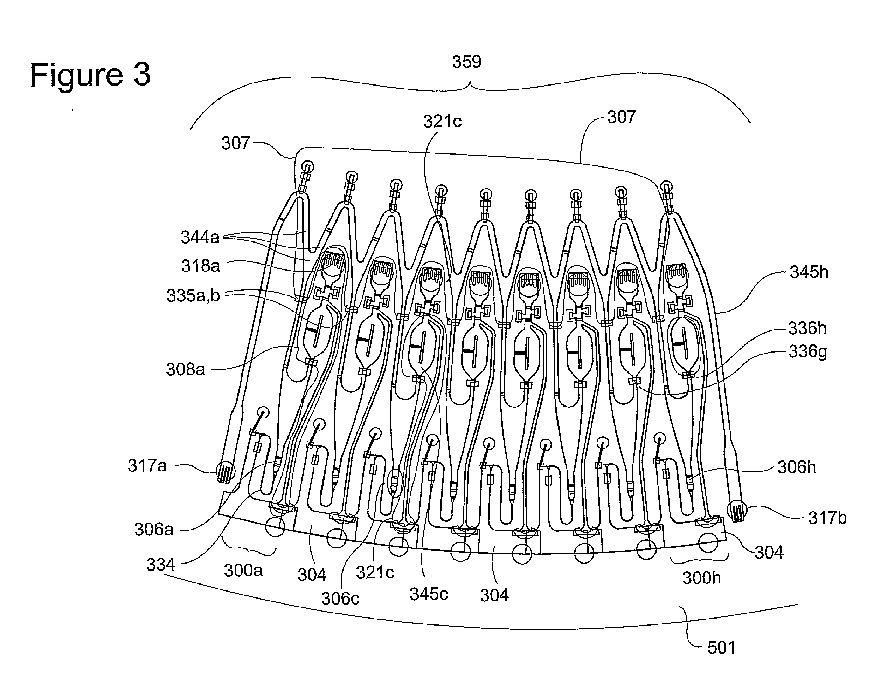

The microfluidic device used for examples 1 and 2 is circular and of the same dimension as a conventional CD (compact disc). The microstructures of the disc are illustrated in figure 3. The device (= CD) contained 14 groups **(359)** (one shown in figure 3) of 8 microchannel structures **(300a-h)** arranged in an annular zone around the center (spin axis) of the disc with a common outlet and waste arrangement (OWA) **(304)** for each group **(359)** close to the periphery. The structures are similar to and function in the same manner as the subgroup illustrated in figures 1-2 of (WO 020 75312, Gyros AB) and the corresponding figures in WO 03024548 (US 20030054563) (Gyros AB) and WO 03024598 (US 20030053934) (Gyros AB).

Each group **(359)** comprises a distribution manifold/inlet unit **(307)** that is common for all eight structures of the group plus one inlet unit **(308a-h)** and one capture microcavity **(306a-h)** per microchannel structure. The common inlet unit **(307)** comprises a) two common inlet ports **(317a-b)** that also will function as outlet ports for excess liquid, and b) one volume-metering microcavity **(344a-h)** for each microchannel structure **(300a-h).** Each of the separate inlet units **(308a-h)** comprises an inlet port **(318a-h)** and a volume-defining unit **(321a-h)** in which there is a volume-metering microcavity **(345a-h).** At the outlet opening of each volume-metering microcavity **(344a-h,345a-h),** there is a passive valve function **(335a-h,336a-h).** For each microchannel structure there is a capture microcavity **(306a-h)** downstream the inlet units **(307,308a-h).** Each capture microcavity **(306a-h)** is in the downstream direction directly connected to a narrow outlet microconduit **(334)** (restriction microconduit). In figure 3 this outlet microconduit **(334)** is designed as an outward bent and is connected to OWA **(304).**

By applying the appropriate volume of aqueous liquid to the inlet port of an inlet unit, capillarity will fill the volume-metering unit(s) connected to the inlet port with liquid. By spinning the disc around its center, liquid can be forced to pass the passive valve **(335a-h,336a-h)** at the outlet of the corresponding volume-metering microcavities for transport through the capture microcavities/solid phases **(306a-h).**

### INSTRUMENTATION

The immunoassay was performed in an automated system. The system (Gyrolab Workstation, prototype 2 instrument equipped with a Laser Induced Fluorescence (LIF) module, Gyros AB, Uppsala, Sweden) was equipped with a CD-spinner, holder for microtiter plates (MTP) and a robotic arm with a holder for 10 capillaries connected to 5 syringe pumps, 2 and 2. Two of the capillaries transferred all the reagents and buffers from a MTP to either of the two common inlet ports **(105a-b)** in the CD. The other eight capillaries transferred individual samples from a MTP to the separate individual inlet ports **(107a-h)** in the CD.

Gyrolab Workstation is a fully automated robotic system controlled by application-specific software. An application specific method within the software controls the spinning of the CD at the precisely controlled speeds and thereby controls the movement of liquids through the microstructures as the application proceeds. Special software was included in order to reduce background noise.

See also WO 02075312 (Gyros AB), WO 03025548 and US 20030054563 (Gyros AB), WO 03025585 and US 200030055576 (Gyros), WO 03056517 and US 200301156763 (Gyros AB) and also www.gyros.com.

### EXPERIMENT 1

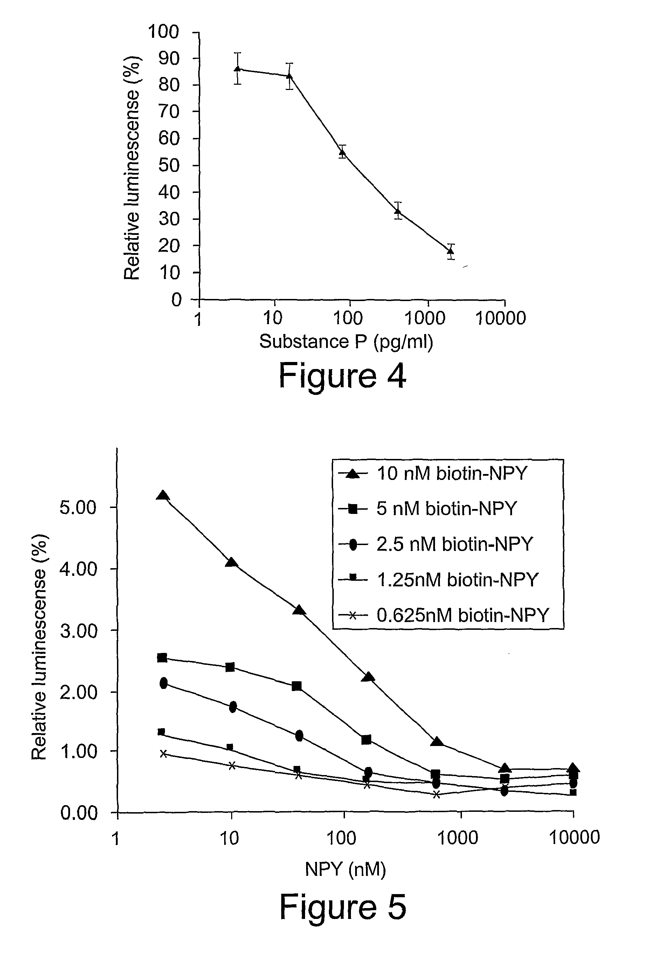

**Assay of Substance P:** Samples containing substance P (0-2000 pg/ml) (10µl; cat.# 80-0206 Assay Designs, Inc. MI, USA) were mixed with substance P-ALP conjugate (10µl; cat.# 80-0266 Assay Designs, Inc. MI, USA) and polyclonal rabbit anti-Substance P (10µl; cat.# 80-0267 Assay Designs, Inc. MI, USA) in microtiter plate (MTP) wells. After incubation at 4 °C overnight, samples (1µl) were withdrawn from the MTP and introduced into the CD via individual inlet ports **(318a-h)** and processed in the instrument given above. Upon spinning of the device [Anti-Substance P/Substance P-ALP]- and [anti-Substance P/Substance P]- complexes were captured in miniaturized affinity columns/capturing microcavity **(306a-h)** in the CD. The affinity columns were created by immobilization of biotinylated goat anti-rabbit IgG (H+L, DS grade, Zymed Laboratories, Inc. CA, USA) on streptavidin coated beads essentially as outlined in WO 04083109 (Gyros AB). The columns were washed four timed with phosphate buffered (pH 7.4) saline containing 0.01% (v/v) Tween® 20. The CD was removed from the instrument, loaded manually with chemiluminescent alkaline phosphatase (ALP) substrate (Lumiphos 530^{™}, cat.# 80-0134 Assay Designs, Inc. MI, USA) and placed on a CD spinner whereupon the columns were equilibrated with the added substrate solution. The intensity of light emitted from the column or from solution that had passed the column was recorded by means of a luminescence detector. Figure 4 shows the resulting data from an assay of substance P standards.

**Figure 4****.** Quantitative assay of substance P performed according to the invented method in a microfluidic device (CD). Substance P standards were mixed with anti-substance P and incubated with substance P standards (0-2000 pg/ml) and Substance P-ALP conjugate. Reaction products were captured in affinity columns (anti-rabbit IgG) in the CD. The columns were equilibrated with chemiluminescent alkaline phosphatase (ALP) substrate (Lumiphos 530^{™}; Lumiphos 530^{™} is a trademark of Lumigen Inc., Southfield, Mi, USA; cat.# 80-0134 Assay Designs, Inc. MI, USA) and the intensity of light emitted from the columns were recorded with a luminescence detector. Samples were analyzed in triplicates.

### EXPERIMENT 2

**Assay of human Neuropeptide Y:** Samples containing human Neuropeptide Y (NPY) (Cat#: 049-03, Phoenix Pharmaceuticals, Inc., CA USA) were mixed with di-biotinyl-lys-NPY (Cat#: B-049-23, Phoenix Pharmaceuticals, Inc., CA USA) and rabbit anti-NPY(Cat#: G-049-03, Phoenix Pharmaceuticals, Inc., CA USA). The reaction mixture contained phosphate buffered saline (PBS) pH 7.4 and 0.1 % (w/v) bovine serum albumin. After incubation in a well of a microtitre plate (MTP) at room temperature the formed product, biotinylated NPY/antibody was captured in the streptavidin coated beads packed as columns in the CD microfluidic device in the same manner as in experiment 1. The column was then treated with Alexa647-labeled goat anti rabbit IgG antibody solution (1: 500 dilution) and subsequently washed with PBS buffer containing 0.01 % (v/v) Tween20. Captured and concentrated biotinylated NPY/antibody complex was quantified by means of a laser induced fluorescence detector. The result for a series of standards is given in **figure 5****.** The CD liquid processing steps and the measurement was performed in the same instrument.

**Figure 5****.** Quantitative assay of human neuropeptide Y according to experiment 2. Anti-NPY (1nM) was mixed and incubated with NPY standards (0-10000 nM) and biotinyl-NPY. Samples were analysed in duplicates.

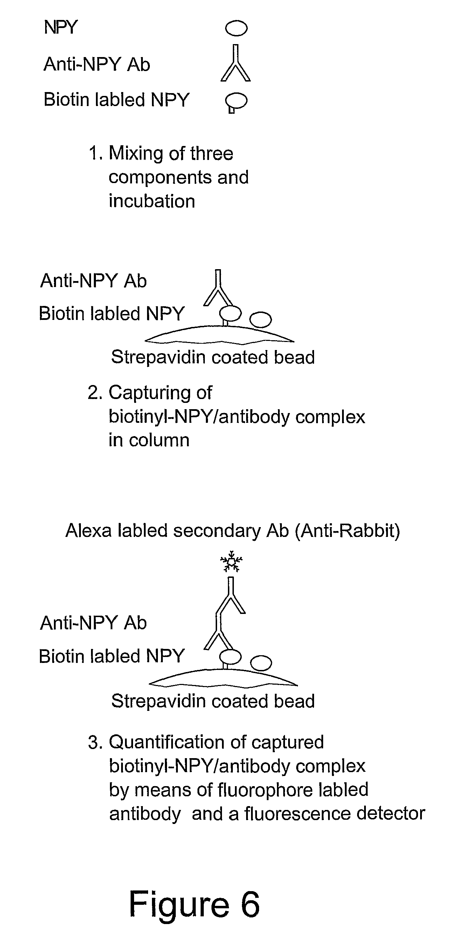

**Figure 6****.** Schematic description of the assay methodology used in experiment 2. First biotinylated analyte (tracer) analyte (sample) and Alexa labelled antibody are mixed and incubated. Subsequently, biotinylated analyte/Alexa labelled antibody complex is captured on streptavidin coated particles packed in a column. Fluorescence-analysis of the column allows for quantification of the biotinylated analyte/Alexa labeled antibody complex.

### EXPERIMENT 3

**Procedure for kinase assay in a CD:** Below follows a schematic description of how kinase assays may be performed in a microfluidic device comprising the microchannel structure given in figure 2 with commercially available reagents. The method is based on the use of a biotinylated substrate that may be a peptide or a protein. The method uses an antibody directed towards a phosphorylated substrate (product P). For tyrosine kinase assays anti-phoshotyrosine specific antibodies are commercially available.
They recognize all phosphorylated tyrosines, regardless of the amino acid surrounding them.

### Kinase activity determination: assay strategy

- Sample: Kinase
- Reagent: Biotinylated peptide (or protein) + Alexa labeled anti-phosphotyrosine (or anti-phospho-serine/threonine) + ATP+Mg²⁺.
- Sample is mixed with reagent and incubated in the reaction microcavity RM (205). Phosphorylated biotinylated product is formed as a result of kinase activity. The phosphorylated product forms a complex with Alexa labeled anti-phosphotyrosine (or serine/threonine).
- The reaction mixture is spun down over the down stream column/solid phase placed in the capturing microcavity **(206).** Biotinylated peptide/Alexa labeled antibody complex is captured in the streptavidin-columns. After column wash, the reaction product can be quantified my means of a LIF detector by integrating the fluorescence signal in the column.

### Kinase inhibitor screening in CD

The proposed assay strategy for kinases described above may also be applicable for inhibitor screening. Importantly, such assays should be possible to perform by mixing only two solutions. Classically the assay principle involves an enzyme-catalysed reaction that is allowed to proceed in the presence of different drug candidates at various concentrations. Each substance is tested at different concentration to determine the inhibition mechanism and inhibition constant. In this type of experiments it is likely that the user has hundreds or thousands of drug candidates (inhibitors) stored as stock solutions in MTP:s. "Small molecule" organic substances are usually dissolved in a water mixable organic solvents such as DMSO or acetonitrile. The "drug" candidates can be considered to be "expensive" reagents as their synthesis often involves many manual steps. Below follows a general assay description.

### Kinase inhibitor screening: assay strategy

- Sample: Inhibitor candidate diluted in ATP + Mg²⁺ solution.
- Reagent: Biotinylated peptide (or protein) + Alexa labeled anti-phosphotyrosine (or anti-phospho-serine/threonine).
- Sample is mixed with reagent and incubated in the upstream mixing chamber in the CD **(231/205).** Phosphorylated biotinylated product is formed as a result of kinase activity. The phosphorylated product forms a complex with Alexa labeled anti-phosphotyrosine (or serine/threonine).
- The reaction mixture is spun down over the down stream column(solid phase/capturing microcavity **(231/205).** Biotinylated peptide/Alexa labeled antibody complex is captured in the streptavidin column. After column wash, the reaction product can be quantified my means of a LIF detector by integrating the fluorescence signal in the column.

Certain innovative aspects of the invention are defined in more detail in the appending claims. Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A microfluidic device comprising one or a plurality of microchannel structures each of which comprises in the downstream direction a reaction microcavity RM (105, 205) and a capture microcavity CM (106, 206) in which there is a solid phase exposing an affinity binder (B), **characterized in that** there are also a) two or more inlet units (108, 109, 110; 207, 208, 209) that are in downstream communication with RM (105, 205) and CM (106, 206), and b) one or more inlet units (107, 111; 210) that are in downstream fluid communication with CM (106, 206) but not with RM (105, 205).

2. The microfluidic device according to claim 1, **characterized in that** there is a mixing function between said two or more inlet units (108, 109, 110; 207, 208, 209) and said RM (105, 205) which mixing function may at least partly coincide with RM (105, 205).

3. The microfluidic device according to claim 1 or 2, **characterized in that** the capture microcavity CM (106, 205) is part of a measuring zone MZ (103) of the microchannel structure, and the reaction microcavity RM (105, 205) is part of a reaction zone RZ (102) upstream of MZ (103).

4. Use of the microfluidic device according to claim 3 for performing a method for determining the amount of an analyte (An) in a sample, wherein the method comprises the steps of:
(i) providing within MZ a product P that has been formed by reacting in a liquid phase An and a reactant (Re) that is capable of binding via affinity to An, wherein the formation of P is part of :
a) a competitive/inhibition affinity assay in which Re is anti-An and P comprises an affinity complex anti-An---An-analogue, or
b) a catalytic assay utilizing a catalytic system that converts a substrate S to P via an affinity complex comprising S and An, where S is Re and An is another component of the catalytic system,
(ii) measuring the amount of P in MZ, and wherein
A) for competitive assays:
(a) An-analogue comprises an immobilizing tag or a group that is transformable to such a tag, and Re comprises a detectable group I, or
(b) An-analogue is a conjugate between an analyte moiety and a detectable group I in the form of a label which label and analyte moiety are linked together via a bridge that preferably is hydrophilic and/or polymeric, and Re comprises an immobilizing tag or a group that is transformable to such a tag, and
B) for catalytic assays substrate S a) comprises an immobilizing tag, or b) is devoid of an immobilizing tag but contains a group that is transformable to such a tag,
C) P is obtained in dissolved form and exhibits the immobilizing tag and the detectable group I,
D) CM contains a predisposed solid phase, and
E) step (ii) comprises the substeps of:
a) immobilizing P via the immobilizing tag to the solid phase, and
b) measuring the amount of immobilized P by measuring detectable group I.

5. The use according to claim 4, wherein
A) the mixing function provides the mixture in which P is to be formed, and
B) step (i) comprises the steps of:
a) introducing and mixing An, Re, and An-analogue and other reactants that are needed for the formation of P into said mixing function in two or more liquid aliquots, the contents of which are such that A) the aliquots differ with respect to kinds of reactant they contain and B) no formation of P can take place without mixing,
b) incubating the mixture in RM, whereafter
c) the mixture is transported through CM.

6. The use according to claim 4 or 5, wherein
A) the measuring zone CM comprises a detection microcavity (DM) that coincides with CM or is fully or partially displaced from CM in the downstream direction, and
B) step (ii.a) is performed in CM and step (ii.b) in DM.

7. The use according to claim 4, 5 or 6, wherein between steps (ii.a) and (ii.b) the solid phase with its immobilized P is separated from remaining soluble forms of An and other reactants including anti-An by transporting the liquid phase downstream within the microchannel structure, possibly by passing one or more aliquots of wash liquid through the solid phase.

8. The use according to any of claims 4 to 7, wherein
A) the microchannel structure comprises an inlet unit that is in downstream communication with CM but not with RZ, and
B) a washing liquid is introduced via this inlet unit and passed through CM.

9. The use according to any of claims 4 to 8, wherein
A) step (i) in claim 4 is according to variant (a),
B) An-analogue and Re are according to (A.a), and
C) step (ii.b) comprises:
a. incorporating into the immobilized affinity complex an affinity counterpart to detectable group I which counterpart contains a detectable group II, for instance a label II, and
b. measuring detectable group. I by measuring detectable group II.

10. The use according to any of claims 4 to 8, wherein
A) step (i) in claim 4 is according to variant (b),
B) the catalytic system is e.g. a biocatalytic system, such as an enzyme system,
C) substrate S is according to (B.a), and
D) An is different from substrate S, for instance is a catalyst, an inhibitor, a co-substrate, a co-catalyst, a co-factor, an analogue to the substrate etc.

11. The use according to any of claims 4 to 8, wherein
a) step (i) is according to variant (b),
b) the catalytic system is e.g. a biocatalytic system, such as an enzyme system,
c) substrate S is according to (B.b), and
d) An is different from substrate S, for instance is a catalyst, an inhibitor, a co-substrate, a co-catalyst, a co-factor, an analogue to the substrate etc.

12. The use according to any of claims 10 to 11 wherein step (ii.b) comprises
A) reacting immobilized P with a reagent that contains
i) a group that is an affinity counterpart to detectable group I, and
ii) a detectable group II, for instance a label II,
to the formation of an affinity complex containing P and this reagent, and
B) measuring detectable group I by measuring detectable group II.

13. The use according to of any of claims 4 to 12, **characterized in that** the amount of Re during incubation in RM is limited, in particular if the use is according to claim 9.

14. The use according to any of claims 4 to 13, wherein
a. the solid phase exposes a reactive counterpart (anti-tag) to the immobilizing tag, said tag defining an affinity immobilizing pair or a covalently immobilizing pair, and
b. P becomes immobilized to the solid phase by interaction between said tag and anti-tag.

15. The use according to any of claims 4 to 14, wherein
A) the micro fluidic device comprises a plurality of said microchannel structures, and
B) at least step (ii.a) is performed in parallel in at least two of said plurality of microchannel structures.
